(19) Europäisches Patentamt
European Patent Office
Office européen des brevets

(11) Veröffentlichungsnummer: **0 166 296**
**B1**

(12) **EUROPÄISCHE PATENTSCHRIFT**

(45) Veröffentlichungstag der Patentschrift: **12.10.88**

(51) Int. Cl.⁴: **C 07 D 211/90**, C 07 D 207/50

(21) Anmeldenummer: **85107174.6**

(22) Anmeldetag: **11.06.85**

# (54) Verfahren zur Herstellung optisch aktiver 1,4-Dihydropyridine.

(30) Priorität: **22.06.84 DE 3423105**

(43) Veröffentlichungstag der Anmeldung: **02.01.86 Patentblatt 86/1**

(45) Bekanntmachung des Hinweises auf die Patenterteilung: **12.10.88 Patentblatt 88/41**

(84) Benannte Vertragsstaaten: **AT BE CH DE FR GB IT LI NL SE**

(56) Entgegenhaltungen: **nichts ermittelt**

(73) Patentinhaber: **BAYER AG, Konzernverwaltung RP Patentabteilung, D-5090 Leverkusen 1 Bayerwerk (DE)**

(72) Erfinder: **Wehinger, Egbert, Dr., Gellertweg 33, D-5600 Wuppertal 1 (DE)**
Erfinder: **Meyer, Horst, Dr., Henselweg 11, D-5600 Wuppertal 1 (DE)**

**EP 0 166 296 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die vorliegende Erfindung betrifft ein neuartiges Verfahren zur Herstellung optisch aktiver 1,4-Dihydropyridin-Derivate.

Es ist bereits bekannt geworden, daß bestimmte 1,4-Dihydropyridin-Derivate interessante pharmakologische Eigenschaften aufweisen und insbesondere als kreislaufbeeinflussende Mittel verwendet werden (vgl. F. Bossert und W. Vater, Naturwissenschaften 58, 578 (1971) und DE-OS-2 117 571).

Weiterhin ist bereits bekannt, daß die Antipoden chiraler 1,4-Dihydropyridin-3,5-dicarbonsäureester nach verschiedenen Verfahren dargestellt werden können (DE-OS-2 935 451; T. Shibanuma et al. Chem. Pharm. Bull. 28, 2809 (1980); DE-OS-3 320 616).

Gegenstand der vorliegenden Erfindung ist ein neues, chemisch eigenartiges Verfahren zur Darstellung der optischen Antipoden chiraler 1,4-Dihydropyridin-3,5-dicarbonsäurediester.

Es wurde gefunden, daß man die teilweise bekannten Antipoden chiraler 1,4-Dihydropyridindicarbonsäureester der allgemeinen Formel (I),

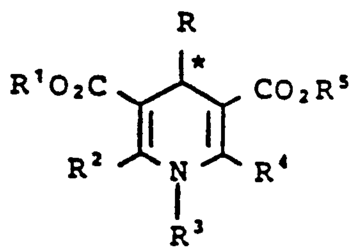

(I)

in welcher

R für einen Phenyl- oder Naphthylrest oder für Thienyl, Furyl, Pyrryl, Pyrazolyl, Imidazolyl, Oxazolyl, Isoxazolyl, Thiazolyl, Pyridyl, Pyridazinyl, Pyrimidyl, Pyrazinyl, Indolyl, Benzimidazolyl, Benzoxazolyl, Benzisoxazolyl, Benzoxadiazolyl, Benzthiadiazolyl, Chinolyl, Isochinolyl, Chinazolyl oder Chinoxalyl steht, wobei die genannten Heterocyclen sowie der Phenylrest und der Naphthylrest 1 bis 3 gleiche oder verschiedene Substituenten tragen können, wobei als Substituenten Phenyl, geradkettiges oder verzweigtes Alkyl mit bis zu 8 Kohlenstoffatomen, Cycloalkyl mit 3 bis 7 Kohlenstoffatomen, Alkenyl oder Alkinyl mit 2 bis 6 Kohlenstoffatomen, Alkoxy mit 1 bis 4 Kohlenstoffatomen, Alkenoxy und Alkinoxy mit 2 bis 6 Kohlenstoffatomen, Tri-, Tetra- und Pentamethylen, Dioxymethylen, Dioxyethyliden, Halogen wie Fluor, Chlor, Brom oder Jod, Trifluormethyl, Trifluorethyl, Trifluormethoxy, Difluormethoxy, Tetrafluorethoxy, Dialkylamino mit 1 bis 4 C-Atomen, Nitro, Cyano, Azido, Alkoxycarbonyl mit 1 bis 4 C-Atomen im Alkoxyrest oder SO -Alkyl, worin m 0 oder 2 bedeutet und Alkyl 1 bis 4 Kohlenstoffatome enthält, aufgeführt seien,

$R^1$ und $R^5$, die immer verschieden sind, für einen achiralen geradkettigen, verzweigten oder cyclischen, gesättigten oder ungesättigten Kohlenwasserstoffrest mit bis zu 8 Kohlenstoffatomen stehen, der gegebenenfalls durch ein Sauerstoff- oder Schwefelatom in der Kette unterbrochen ist und/oder der gegebenenfalls substituiert ist durch Halogen wie Fluor oder Chlor, Cyano, Alkoxycarbonyl mit bis zu 4 Kohlenstoffatomen im Alkylteil, Phenyl, Phenoxy, Phenylthio oder Phenylsulfonyl, wobei die Phenylgruppen ihrerseits substituiert sein können durch Halogen wie Fluor oder Chlor, Cyano, Dialkylamino mit bis zu 4 Kohlenstoffatomen je Alkylgruppe, Alkoxy oder Alkyl mit jeweils bis zu 4 Kohlenstoffatomen, Trifluormethyl oder Nitro, oder wobei der Kohlenwasserstoffrest substituiert sein kann durch Pyridyl oder eine Aminogruppe, wobei diese Aminogruppe durch zwei gleiche oder verschiedene Substituenten aus der Gruppe Alkyl mit bis zu 4 Kohlenstoffatomen, Alkoxyalkyl mit bis zu 6 Kohlenstoffatomen, Aryl, insbesondere Phenyl, oder Aralkyl, insbesondere Benzyl, substituiert ist, oder wobei die Aminogruppe in der Weise substituiert ist, daß zwei Reste gemeinsam mit dem Stickstoffatom einen 5- bis 7-gliedrigen heterocyclischen Ring bilden, der als weiteres Heteroatom Sauerstoff oder Schwefel oder eine N-Alkyl/Phenyl-Gruppierung enthalten kann, und

$R^1$ und $R^4$, gleich oder verschieden sein können, für Wasserstoff oder vorzugsweise für einen geradkettigen oder verzweigten, gegebenenfalls substituierten Alkylrest mit bis zu 4 Kohlenstoffatomen, einen Phenyl- oder einen Benzylrest stehen, und

$R^3$ vorzugsweise für Wasserstoff oder einen achiralen geradkettigen oder verzweigten Alkylrest mit bis zu 4 Kohlenstoffatomen, einen Phenylrest oder einen Benzylrest steht,

erhält, wenn man die Verbindungen der allgemeinen Formel (II) (einschließlich ihrer prototropen Formen)

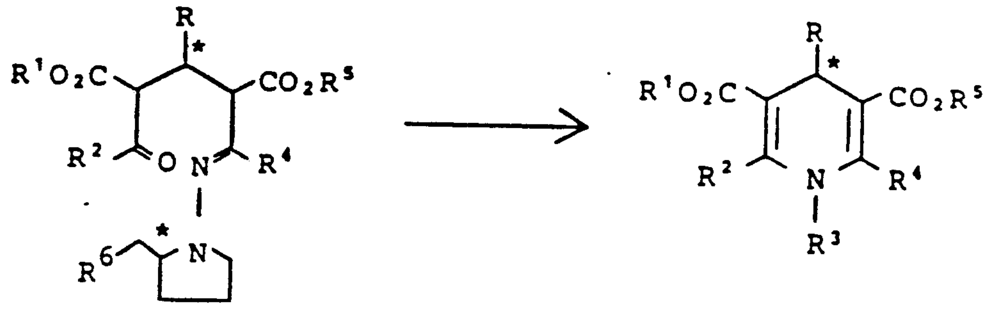

II → I

in welcher die Reste R, $R^1$, $R^2$ $R^4$ und $R^5$ die oben angegebene Bedeutung haben und $R^6$ für einen Alkyl- oder Aralkylrest steht und in welcher die Konfiguration an den mit (*) bezeichneten Kohlenstoffatomen einheitlich definiert ist,

mit Aminen der allgemeinen Formel (III)

$$R^3\text{-}NH_2 \qquad (III)$$

bzw. deren Säureadditionssalzen,

in welchen $R^3$ die oben angegebene Bedeutung hat,

gegebenenfalls in Gegenwart geeigneter Lösungsmittel zur Reaktion bringt.

Ein wesentlicher Vorteil des Verfahrens liegt darin, daß es aufgrund seiner einfachen Reaktionsbedingungen mit geringem technischen Aufwand und hoher Wirtschaftlichkeit durchgeführt werden kann, wobei besonders darauf hinzuweisen ist, daß bei der Umsetzung (S) - bzw. (R)-1-Amino-2-alk(aralk)oxymethyl-pyrrolidin wieder freigesetzt wird und erneut als chiraler Induktor in den Reaktionsprozeß eingebracht werden kann.

Die Darstellung eines optisch aktiven 1,4-Dihydro-2,6-dimethyl-4-(3-nitrophenyl)-pyridin-3,5-dicarbonsäure-ethyl-isopropyl-esters kann beispielhaft durch folgendes Formelschema wiedergegeben werden:

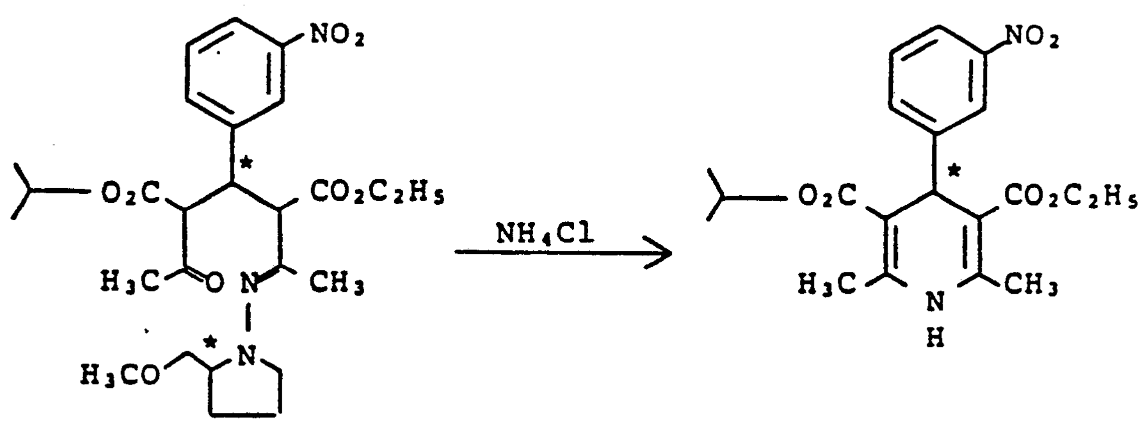


Nach dem erfindungsgemäßen Verfahren erhält man ein optisch aktives 1,4-Dihydropyridin-Derivat der allgemeinen Formel (I) durch Umsetzung eines optisch aktiven, einheitlich konfigurierten Hydrazons der allgemeinen Formel (II) mit einem Amin bzw. dessen Säureadditionssalz der allgemeinen Formel (III) gegebenenfalls in Gegenwart eines geeigneten Lösungsmittels.

In der Formel II steht $R^6$ vorzugsweise für einen achiralen geradkettigen oder verzweigten Alkylrest mit bis zu 4 Kohlenstoffatomen, insbesondere für die Methylgruppe, oder für einen Aralkylrest, insbesondere für den Benzylrest.

Die Verbindungen der allgemeinen Formel (II) sind neu und können in Analogie zu literaturbekannten Verfahren durch Addition eines optisch aktiven Hydrazons des (S)-bzw. (R)-1-Amino-2-alk(aralk)oxymethyl-pyrrolidins der allgemeinen Formel (IV) einschließlich seiner prototropen Formen an stereochemisch einheitliche

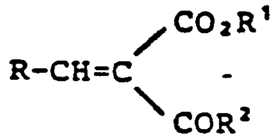

V

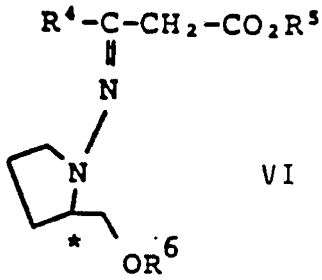

VI

prochirale Yliden-ß-ketocarbonsäureester der allgemeinen Formel (V) erhalten werden (vgl. D, Enders und K. Papdopoulos, Tetrahedron Letters 24, 4967 (1983)). In den Formeln (IV) und (V) haben die Reste R, $R^1$, $R^2$, $R^4$, $R^5$ und $R^6$ die oben angegebene Bedeutung.

Die als Ausgangsverbindungen eingesetzten Amine der Formel (III) bzw. deren Säureadditionssalze sind bekannt. Als Säureadditionssalze kommen die Salze sowohl von anorganischen als auch von organischen Säuren in Frage, wobei Halogenwasserstoffsäuren, Schwefelsäure, Kohlensäure und Essigsäure beispielhaft genannt seien.

Insbesondere seien genannt Ammonium- und Methylammonium-Sulfate, Chloride, Carbonate, Hydrogencarbonate, Acetate und Oxalate.

Als Verdünnungsmittel können alle inerten organischen Lösungsmittel Verwendung finden. Hierzu gehören vorzugsweise Alkohole wie Methanol oder Ethanol, Ether wie Tetrahydrofuran, Glykolmonomethylether oder Glykoldimethylether, Eisessig, Dimethylformamid, Dimethylsulfoxid, Acetonitril, Pyridin oder Hexamethylphosphorsäuretriamid.

Die Reaktionstemperaturen können in einem größeren Bereich variiert werden. Im allgemeinen arbeitet man zwischen 0 und 150° C, vorzugsweise zwischen 20 und 100° C, insbesondere bei Siedetemperatur des jeweiligen Lösungsmittels.

Die Umsetzung kann bei Normaldruck, aber auch bei erhöhtem Druck durchgeführt werden. Im allgemeinen arbeitet man unter Normaldruck.

Bei der Durchführung des erfindungsgemäßen Verfahrens wird 1 Mol des Hydrazons der allgemeinen Formel (II) mit 1 bis 5 Mol des Amins der allgemeinen Formel (III) bzw. dessen Säureadditionssalzes in einem geeigneten Lösungsmittel zur Reaktion gebracht.

Die Isolierung und Reinigung der Endprodukte erfolgt vorzugsweise derart, daß man das Lösungsmittel im Vakuum abdestilliert und den Rückstand gegebenenfalls den aus dem Stand der Technik her bekannten Reinigungsoperationen wie der Umkristallisation aus einem geeigneten Lösungsmittel oder der chromatographischen Trennung unterwirft.

Das erfindungsgemäße Verfahren eignet sich zur Darstellung der optischen Antipoden chiraler 1,4-Dihydropyridin-3,5-dicarbonsäurediester mit einem breiten Spektrum von Substituenten und Strukturvariationen.

Außer den unten angeführten Herstellungsbeispielen seien die Enantiomere folgender Wirkstoffe besonders erwähnt:

1,4-Dihydro-2,6-dimethyl-4-(3-nitrophenyl)-pyridin-3,5-dicarbonsäure-isobutyl-methyl-ester,
1,4-Dihydro-2,6-dimethyl-4-(3-nitrophenyl)-pyridin-3,5-dicarbonsäure-methyl-neopentylester,
1,4-Dihydro-2,6-dimethyl-4-(3-nitrophenyl)-pyridin-3,5-dicarbonsäure-cyclopentyl-methyl-ester,
1,4-Dihydro-2,6-dimethyl-4-(3-nitrophenyl)-pyridin-3,5-dicarbonsäure-isopropyl-(2-methoxyethyl)-ester,
1,4-Dihydro-2,6-dimethyl-4-(3-nitrophenyl)-pyridin-3,5-dicarbonsäure-methyl-(2-N-benzyl-N-methylaminoethyl)-ester,
1,4-Dihydro-2,6-dimethyl-4-(3-nitrophenyl)-pyridin-3,5-dicarbonsäure-methyl-(2-(4-phenyl-piperazinyl-1)-ethyl)-ester,
1,4-Dihydro-2,6-dimethyl-4-(2-nitrophenyl)-pyridin-3,5-dicarbonsäure-benzyl-methyl-ester,
1,4-Dihydro-2,6-dimethyl-4-(2-nitrophenyl)-pyridin-3,5-dicarbonsäure-methyl-(2-phenoxyethyl)-ester,
1,4-Dihydro-2,6-dimethyl-4-(2-trifluormethylphenyl)-pyridin-3,5-dicarbonsäure-isopropyl-methyl-ester,
1,4-Dihydro-2,6-dimethyl-4-(2-chlorphenyl)-pyridin-3,5-dicarbonsäure-allyl-methyl-ester,
1,4-Dihydro-2,6-dimethyl-4-(2,3-dichlorphenyl)-pyridin-3,5-dicarbonsäure-ethyl-methyl-ester,
1,4-Dihydro-2,6-dimethyl-4-(2,3-dichlorphenyl)-pyridin-3,5-dicarbonsäure-hexyl-methyl-ester,
1,4-Dihydro-2,6-dimethyl-4-(2-chlorphenyl)-pyridin-3,5-dicarbonsäure-methyl-(2,2,2-trifluorethyl)-ester,
1,4-Dihydro-2,6-dimethyl-4-(2-Chlorphenyl)-pyridin-3,5-dicarbonsäure-ethyl-(2,2,2-trifluorethyl)-ester,
1,4-Dihydro-2,6-dimethyl-4-(2-cyanophenyl)-pyridin-3,5-dicarbonsäure-ethyl-methyl-ester,
1,4-Dihydro-2,6-dimethyl-4-(pyridyl-3)-pyridin-3,5-dicarbonsäure-ethyl-methyl-ester,

1,4-Dihydro-2,6-dimethyl-4-(pyridyl-2)-pyridin-2,5-dicarbonsäure-cyclohexyl-methyl-ester,
1,4-Dihydro-2,6-dimethyl-4-(2,1,3-benzoxadiazolyl-4)-pyridin-3,5-dicarbonsäure-isopropyl-methyl-ester,
1,4-Dihydro-2,6-dimethyl-4-(2,1,3-benzoxadiazolyl-4)-pyridin-3,5-dicarbonsäure-ethyl-methyl-ester,
1,4-Dihydro-1,2,6-trimethyl-4-(2,1,3-benzoxadiazolyl-4)-pyridin-3,5-dicarbonsäure-isopropyl-methyl-ester,
1,4-Dihydro-1,2,6-trimethyl-4-(3-nitrophenyl)-pyridin-3,5-dicarbonsäure-ethyl-methyl-ester,
1,4-Dihydro-1,2,6-trimethyl-4-(2-nitrophenyl)-pyridin-3,5-dicarbonsäure-isobutyl-methyl-ester.

## Herstellungsbeispiele

**Beispiel 1**

(-)-1,4-Dihydro-2,6-dimethyl-4-(3-nitrophenyl)-pyridin-3,5-dicarbonsäure-ethyl-methyl-ester

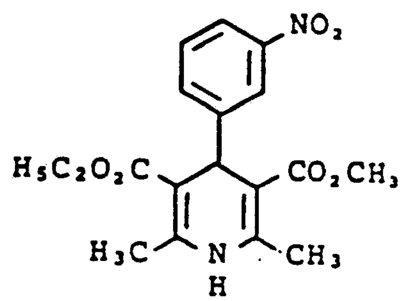

(-)

Zu einer Lösung von 10 g (43,8 mMol) (S)-3-[(2-Methoxymethylpyrrolidin-1-yl)-imino]-buttersäuremethylester in 90 ml absolutem Tetrahydrofuran wurden nach Zugabe von 25 ml Tetramethylethylendiamin bei -70° C und unter Stickstoff 28 ml (44,8 mMol) einer 1,6 molaren BuLi-Lösung in n-Hexan zugetropft. Anschließend wurde bei -70° C eine Lösung von 11,5 g (43,8 mMol) 2-(3-Nitrobenzyliden)-acetessig-säureethylester in 75 ml Tetrahydrofuran zugetropft. Nach einstündigem Rühren bei -70° C wurde das Kühlbad entfernt, die Reaktionslösung bis zum Erreichen von Zimmertemperatur sich selbst überlassen (ca. 3 bis 4 Stunden) und anschließend vorsichtig in Ether/Wasser gegossen. Die wäßrige Phase wurde erneut mit Ether extrahiert und die vereinigten etherischen Extrakte nach Trocknen über wasserfreiem Natriumsulfat im Vakuum eingeengt, wobei ein öliger Rückstand (15 g M⊕ = 491) resultierte, der chromatographisch gereinigt wurde. 12 g (24 mMol) dieses Zwischenproduktes wurden in 50 ml Methanol aufgenommen und nach Zugabe von 3,2 g (60 mMol) Ammoniumchlorid 15 Stunden zum Sieden erhitzt. Anschließend wurde das Lösungsmittel im Vakuum abdestilliert, der Rückstand in Dichlormethan aufgenommen und mit Wasser gewaschen. Die organische Phase wurde nach Trocknen über $NaSO_4$ eingeengt. Das rückbleibende Öl kristallisierte nach Verreiben mit Ether teilweise durch, das Rohprodukt wurde abgesaugt, mit Ether gewaschen und getrocknet, 3,3 g (38 %), Fp: 159° C.

$[\alpha]^{20}_D$ = -14,97° (C = 0,57 %, Ethanol).

**Beispiel 2**

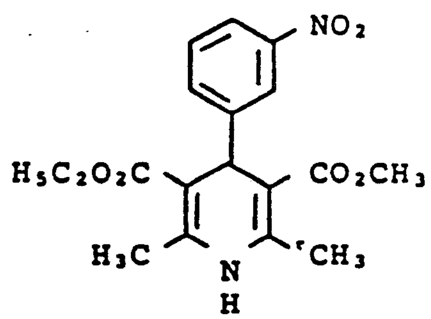

(+)

Analog Beispiel 1 wurde unter Verwendung des (R)-3-[(2-Methoxymethylpyrrolidin-1-yl)-imino]-buttersäure-methylesters der rechsdrehende 1,4-Dihydro-2,6-dimethyl-4-(3-nitrophenyl)-pyridin-3,5-dicarbonsäure-ethyl-

methylester erhalten.
Fp: 160° C,
$[\alpha]^{20}_D$ = +15,56 (= 0,41 %, Ethanol).

**Beispiel 3**

(-)-1,4-Dihydro-2,6-dimethyl-4-(3-nitrophenyl)-pyridin-3,5-dicarbonsäure-isopropyl-methyl-ester

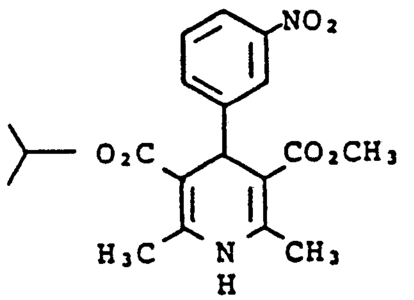

(-)

Zu einer Lösung von 7,1 g (31 mMol) (S)-3-[(2-Methoxymethylpyrrolidin-1-yl)-imino]-buttersäuremethylester in 60 ml absolutem Tetrahydrofuran wurden nach Zugabe von 20 ml Tetramethylethylendiamin bei -70° C und unter Stickstoff 20 ml (32 mMol) einer 1,6 molaren Lösung von Butyllithium in n-Hexan zugetropft. Anschließend wurde bei -70° C eine Lösung von 8,6 g (31 mMol) 2-(3-Nitrobenzyliden)-acetessigsäureisopropylester in 40 ml Tetrahydrofuran zugetropft. Die Reaktionsmischung wurde 1 Stunde bei -70° C gerührt, anschließend wurde das Kühlbad entfernt und nach Erreichen von Zimmertemperatur (ca. 3 bis 4 Stunden) wurde die Lösung langsam in Ether/Wasser gegossen. Die wäßrige Phase wurde mehrmals mit Ether extrahiert und die vereinigten organischen Extrakte über wasserfreiem Natriumsulfat getrocknet. Verdampfen 0 des Lösungsmittels unter Vakuum lieferte ein öliges Zwischenprodukt (12 g, M⊕ = 505), das chromatographisch gereinigt wurde. 8 g (15,8 mMol) dieser Verbindung wurden in 50 ml Methanol gelöst und nach Zugabe von 3,4 g (64 mMol) Ammoniumchlorid 16 Stunden unter Rückfluß erhitzt. Das Lösungsmittel wurde im Vakuum abdestilliert, der Rückstand in Dichlormethan aufgenommen und mit Wasser gewaschen. Die organische Phase wurde über wasserfreiem Natriumsulfat getrocknet, unter Vakuum eingeengt und der ölige Rückstand durch Verreiben mit wenig Ether zur Kristallisation gebracht. Das Festprodukt wurde abgesaugt und getrocknet, 2,3 g (39 %), Fp: 136° C
$[\alpha]^{20}_D$ = -24,60° (C = 1,07 %, Ethanol).

**Beispiel 4**

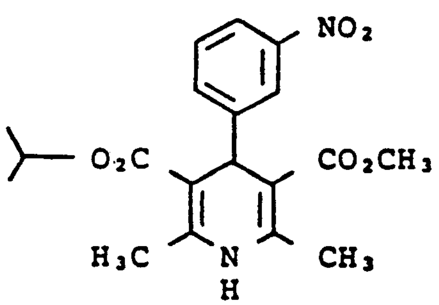

(+)

Analog Beispiel 3 wurde unter Verwendung des (R)-3-[(2-Methoxymethylpyrrolidon-1-yl)-imino]-buttersäuremethylesters der rechtsdrehende 1,4-Dihydro-2,6-dimethyl-4-(3-nitrophenyl)-pyridin-3,5-dicarbonsäure-isopropyl-methyl-ester erhalten.
Fp: 136° C
$[\alpha]^{20}_D$ = +24,97° (C = 0,93 %, Ethanol).

**Beispiel 5**

(-)-1,4-Dihydro-2,6-dimethyl-4-(2-nitrophenyl)-pyridin-3,5-dicarbonsäure-isobutyl-methyl-ester

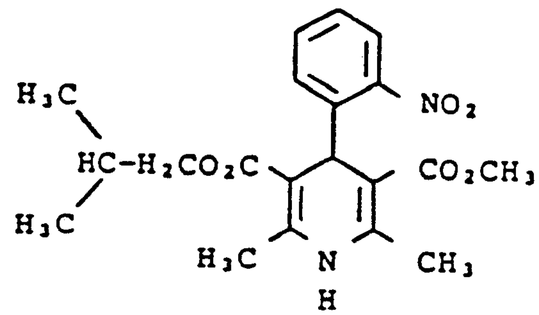

(-)

Zu einer Lösung von 7,1 g (31 mMol) (S)-3-[(2-Methoxymethylpyrrolidin-1-yl)-imino]-buttersäuremethylester in 60 ml absolutem Tetrahydrofuran wurden nach Zugabe von 20 ml Tetramethylethylendiamin bei -70° C und unter Stickstoff 20 ml (32 mMol) einer 1,6 molaren Lösung von Butyllithium in n-Hexan zugetropft. Anschließend wurde bei -70°C eine Lösung von 9 g (31 mMol) 2-(2-Nitrobenzyliden)-acetessigsäureisobutylester in 50 ml Tetrahydrofuran zugetropft und die Lösung noch 1 Stunde bei -70°C gerührt. Das Kühlbad wurde entfernt und nach Erreichen von Zimmertemperatur (ca. 3 bis 4 Stunden) wurde die Lösung langsam in Ether/Wasser gegossen. Die wäßrige Phase wurde nochmals mit Ether extrahiert und die vereinigten organischen Extrakte über wasserfreiem Natriumsulfat getrocknet. Abdestillieren des Lösungsmittels unter Vakuum ergab ein öliges Zwischenprodukt (12,8 g, $M^{\oplus}$ = 519), das chromatographisch über Kieselgel gereinigt wurde. 6,4 g (12,3 mMol) dieser Verbindung wurden in 25 ml Methanol gelöst und nach Zugabe von 1,61 g (30,2 mMol) Ammoniumchlorid 15 Stunden unter Rückfluß erhitzt. Das Lösungsmittel wurde im Vakuum abdestilliert, der Rückstand in Dichlormethan aufgenommen und mit Wasser gewaschen. Die organische Phase wurde über wasserfreiem Natriumsulfat getrocknet, unter Vakuum eingeengt und der ölige Rückstand chromatographisch gereinigt, 1,4 g (30 %) amorphes Produkt.

$[\alpha]^{20}_D$ = -166,4° (0,49 % w/v, Ethanol).

**Beispiel 6**

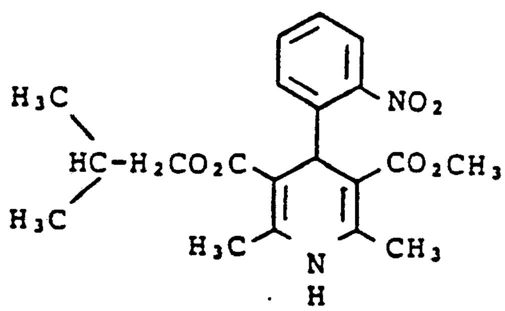

(+)

Analog Beispiel 5 wurde unter Verwendung des (R)-3-[(2-Methoxymethylpyrrolidin-1-yl)-imino]-buttersäuremethylesters der rechtsdrehende 1,4-Dihydro-2,6-dimethyl-4-(2-nitrophenyl)-pyridin-3,5-dicarbonsäure-isobutyl-methylester erhalten.

$[\alpha]^{20}_D$ = +166,3° (0,68 % w/v, Ethanol).

**Patentansprüche**

1. Verfahren zur Herstellung von Antipoden chiraler 1,4-Dihydropyridin-dicarbonsäureester der allgemeinen Formel (I),

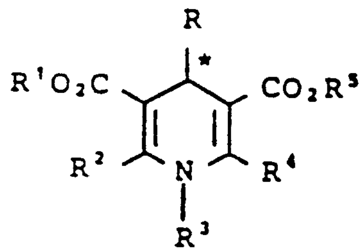

(I)

in welcher
R für einen Phenyl- oder Naphthylrest oder für Thienyl, Furyl, Pyrryl, Pyrazolyl, Imidazolyl, Oxazolyl, Isoxazolyl, Thiazolyl, Pyridyl, Pyridazinyl, Pyrimidyl, Pyrazinyl, Indolyl, Benzimidazolyl, Benzoxazolyl, Benzisoxazolyl, Benzoxadiazolyl, Benzthiadiazolyl, Chinolyl, Isochinolyl, Chinazolyl oder Chinoxalyl, wobei die genannten Heterocyclen sowie der Phenylrest und der Naphthylrest 1 bis 3 gleiche oder verschiedene Substituenten tragen können, wobei als Substituenten Phenyl, geradkettiges oder verzweigtes Alkyl mit bis zu 8 Kohlenstoffatomen, Cycloalkyl mit 3 bis 7 Kohlenstoffatomen, Alkenyl oder Alkinyl mit 2 bis 6 Kohlenstoffatomen, Alkoxy mit 1 bis 4 Kohlenstoffatomen, Alkenoxy und Alkinoxy mit 2 bis 6 Kohlenstoffatomen, Tri-, Tetra- und Pentamethylen, Dioxymethylen, Dioxyethyliden, Halogen wie Fluor, Chlor, Brom oder Jod, Trifluormethyl, Trifluorethyl, Trifluormethoxy, Difluormethoxy, Tetrafluorethoxy, Dialkylamino mit 1 bis 4 C-Atomen, Nitro, Cyano, Azido, Alkoxycarbonyl mit 1 bis 4 C-Atomen im Alkoxyrest oder $SO_m$-Alkyl, worin m 0 oder 2 bedeutet und Alkyl 1 bis 4 Kohlenstoffatome enthält, aufgeführt seien,
$R^1$ und $R^5$, die immer verschieden sind, für einen achiralen geradkettigen, verzweigten oder cyclischen, gesättigten oder ungesättigten Kohlenwasserstoffrest mit bis zu 8 Kohlenstoffatomen, der gegebenenfalls durch ein Sauerstoff- oder Schwefelatom in der Kette unterbrochen ist und/ oder der gegebenenfalls substituiert ist durch Halogen wie Fluor oder Chlor, Cyano, Alkoxycarbonyl mit bis zu 4 Kohlenstoffatomen im Alkylteil, Phenyl, Phenoxy, Phenylthio oder Phenylsulfonyl, wobei die Phenylgruppen ihrerseits substituiert sein können durch Halogen wie Fluor oder Chlor, Cyano, Dialkylamino mit bis zu 4 Kohlenstoffatomen je Alkylgruppe, Alkoxy oder Alkyl mit jeweils bis zu 4 Kohlenstoffatomen, Trifluormethyl oder Nitro, oder wobei der Kohlenwasserstoffrest substituiert sein kann durch Pyridyl oder eine Aminogruppe, wobei diese Aminogruppe durch zwei gleiche oder verschiedene Substituenten aus der Gruppe Alkyl mit bis zu 4 Kohlenstoffatomen, Alkoxyalkyl mit bis zu 6 Kohlenstoffatomen, Aryl, insbesondere Phenyl, oder Aralkyl, insbesondere Benzyl, substituiert ist, oder wobei die Aminogruppe in der Weise substituiert ist, daß zwei Reste gemeinsam mit dem Stickstoffatom einen 5- bis 7-gliedrigen heterocyclischen Ring bilden, der als weiteres Heteroatom Sauerstoff oder Schwefel oder eine N-Alkyl/Phenyl-Gruppierung enthalten kann,
$R^2$ und $R^4$, gleich oder verschieden sein können, für Wasserstoff oder vorzugsweise für einen geradkettigen oder verzweigten, gegebenenfalls substituierten Alkylrest mit bis zu 4 Kohlenstoffatomen, einen Phenyloder einen Benzylrest, und
$R^3$ für Wasserstoff oder einen achiralen geradkettigen oder verzweigten Alkylrest mit bis zu 4 Kohlenstoffatomen, einen Phenylrest oder einen Benzylrest steht,
dadurch gekennzeichnet, daß man Verbindungen der allgemeinen Formel (II) (einschließlich ihrer prototropen Formen)

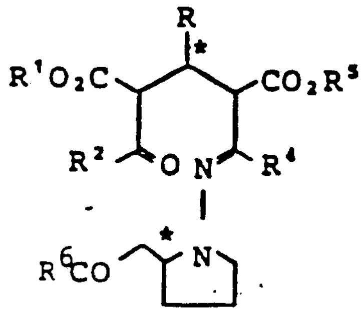

II

in welcher die Reste R, $R^1$, $R^2$, $R^4$ und $R^5$ die oben angegebene Bedeutung haben und $R^6$ für einen Alkyl- oder Aralkylrest steht und in welcher die Konfiguration an den mit (*) bezeichneten Kohlenstoffatomen einheitlich definiert ist,

mit aminen der allgemeinen Formel (III)

$R^3$-$NH_2$ (III)

bzw. deren Säureadditionssalzen,
in welchen $R^3$ die oben angegebene Bedeutung hat,
gegebenenfalls in Gegenwart geeigneter Lösungsmittel zur Reaktion bringt.

2. Verfahren gemäß Anspruch 1, dadurch gekennzeichnet, daß man Säureadditionssalze der Verbindungen der allgemeinen Formel (III) aus der Gruppe Ammonium- und Methylammonium-Sulfate, Chloride, Carbonate, Hydrogencarbonate, Acetate oder Oxalate verwendet.

3. Verfahren gemäß Ansprüchen 1 und 2, dadurch gekennzeichnet, daß man die ümsetzung bei Temperaturen zwischen 0 und 150° C und in Gegenwart eines inerten organischen Lösungsmittels durchführt.

4. Verfahren gemäß Ansprüchen 1 bis 3, dadurch gekennzeichent, daß man die optischen Antipoden der allgemeinen Formel (I) chromatographisch reinigt.

5. Verbindungen der allgemeinen Formel (II)

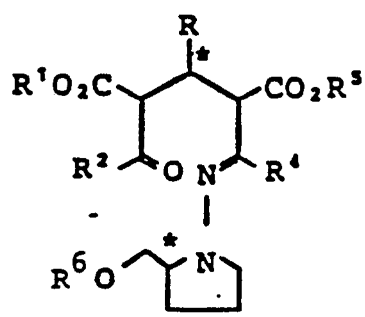

(II)

in welcher

R für einen Phenyl- oder Naphthylrest oder für Thienyl, Furyl, Pyrryl, Pyrazolyl, Imidazolyl, Oxazolyl, Isoxazolyl, Thiazolyl, Pyridyl, Pyridazinyl, Pyrimidyl, Pyrazinyl, Indolyl, Benzimidazolyl, Benzoxazolyl, Benzisoxazolyl, Benzoxadiazolyl, Benzthiadiazolyl, Chinolyl, Isochinolyl, Chinazolyl oder Chinoxalyl, wobei die genannten Heterocyclen sowie der Phenylrest und der Naphthylrest 1 bis 3 gleiche oder verschiedene Substituenten tragen können, wobei als Substituenten Phenyl, geradkettiges oder verzweigtes Alkyl mit bis zu 8 Kohlenstoffatomen, Cycloalkyl mit 3 bis 7 Kohlenstoffatomen, Alkenyl oder Alkinyl mit 2 bis 6 Kohlenstoffatomen, Alkoxy mit 1 bis 4 Kohlenstoffatomen, Alkenoxy und Alkinoxy mit 2 bis 6 Kohlenstoffatomen, Tri-, Tetra- und Pentamethylen, Dioxymethylen, Dioxyethyliden, Halogen wie Fluor, Chlor, Brom oder Jod, Trifluormethyl, Trifluorethyl, Trifluormethoxy, Difluormethoxy, Tetrafluorethoxy, Dialkylamino mit 1 bis 4 C-Atomen, Nitro, Cyano, Azido, Alkoxycarbonyl mit 1 bis 4 C-Atomen im Alkoxyrest oder $SO_m$-Alkyl, worin m 0 oder 2 bedeutet und Alkyl 1 bis 4 Kohlenstoffatome enthält, aufgeführt seien,

$R^1$ und $R^5$, die immer verschieden sind, für einen achiralen geradkettigen, verzweigten oder cyclischen, gesättigten oder ungesättigten Kohlenwasserstoffrest mit bis zu 8 Kohlenstoffatomen, der gegebenenfalls

durch ein Sauerstoff- oder Schwefelatom in der Kette unterbrochen ist und/oder der gegebenenfalls substituiert ist durch Halogen wie Fluor oder Chlor, Cyano, Alkoxycarbonyl mit bis zu 4 Kohlenstoffatomen im Alkylteil, Phenyl, Phenoxy, Phenylthio oder Phenylsulfonyl, wobei die Phenylgruppen ihrerseits substituiert sein können durch Halogen wie Fluor oder Chlor, Cyano, Dialkylamino mit bis zu 4 Kohlenstoffatomen je Alkylgruppe, Alkoxy oder Alkyl mit jeweils bis zu 4 Kohlenstoffatomen, Trifluormethyl oder Nitro, oder wobei der Kohlenwasserstoffrest substituiert sein kann durch Pyridyl oder eine Aminogruppe, wobei diese Aminogruppe durch zwei gleiche oder verschiedene Substituenten aus der Gruppe Alkyl mit bis zu 4 Kohlenstoffatomen, Alkoxyalkyl mit bis zu 6 Kohlenstoffatomen, Aryl, insbesondere Phenyl, oder Aralkyl, insbesondere Benzyl, substituiert ist, oder wobei die Aminogruppe in der Weise substituiert ist, daß zwei Reste gemeinsam mit dem Stickstoffatom einen 5- bis 7-gliedrigen heterocyclischen Ring bilden, der als weiteres Heteroatom Sauerstoff oder Schwefel oder eine N-Alkyl/Phenyl-Gruppierung enthalten kann,

$R^2$ und $R^4$, gleich oder verschieden sein können, für Wasserstoff oder vorzugsweise für einen geradkettigen oder verzweigten, gegebenenfalls substituierten Alkylrest mit bis zu 4 Kohlenstoffatomen, einen Phenyl- oder einen Benzylrest, und

$R^6$ für einen achiralen geradkettigen oder verzweigten Alkylrest mit bis zu 4 Kohlenstoffatomen, insbesondere für die Methylgruppe oder für einen Aralkylrest, insbesondere für den Benzylrest,

stehen.

6. Verfahren zur Herstellung von Verbindungen der allgemeinen Formel (II) gemäß Anspruch 5, dadurch gekennzeichnet, daß man ein optisch aktives Hydrazon der allgemeinen Formel (IV)

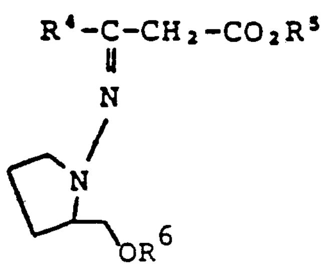

(IV)

in welcher

$R^4$, $R^5$ und $R^6$ die im Anspruch 6 angegebene Bedeutung haben,

mit stereochemisch einheitlichen prochiralen Yliden-β-ketocarbonsäureestern der allgemeinen Formel (V)

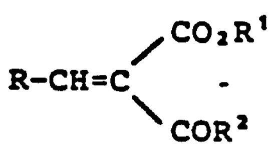

(V)

in welcher

R,$R^1$ und $R^2$ die in Anspruch 6 angegebene Bedeutung haben,

gegebenenfalls in Gegenwart von inerten organischen Lösungsmitteln, umsetzt.

7. Verwendung von Verbindungen der allgemeinen Formel (II) gemäß Anspruch 5, bei der Herstellung von Verbindungen der allgemeinen Formel (I) gemäß Anspruch 1.

**Claims**

1. Process for the preparation of antipodes of chiral 1,4-dihydropyridine-dicarboxylic acid esters of the general formula (I)

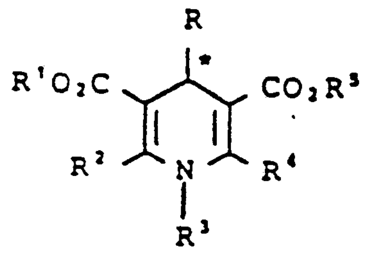

(I)

in which

R represents a phenyl or naphthyl radical, or represents thienyl, furyl, pyrryl, pyrazolyl, imidazolyl, oxazolyl, isoxazolyl, thiazolyl, pyridyl, pyridazinyl, pyrimidyl, pyrazinyl, indolyl, benzimidazolyl, benzoxazolyl, benzisoxazolyl, benzoxadiazolyl, benzothiadiazolyl, quinolyl, isoquinolyl, quinazolyl or quinoxalyl, it being possible for the heterocyclic radicals mentioned and the phenyl radical and naphthyl radical to carry 1 to 3 identical or different substituents, substituents which may be mentioned being phenyl, straight-chain or branched alkyl with up to 8 carbon atoms, cycloalkyl with 3 to 7 carbon atoms, alkenyl or alkinyl with 2 to 6 carbon atoms, alkoxy with 1 to 4 carbon atoms, alkenoxy and alkinoxy with 2 to 6 carbon atoms, tri-, tetra- and penta-methylene, dioxymethylene, dioxyethylidene, halogen, such as fluorine, chlorine, bromine or iodine, trifluoromethyl, trifluoroethyl, trifluoromethoxy, difluoromethoxy, tetrafluoroethoxy, dialkylamino with 1 to 4 C atoms, nitro, cyano, azido, alkoxycarbonyl with 1 to 4 C atoms in the alkoxy radical and $SO_m$-alkyl, m denoting 0 or 2 and alkyl containing 1 to 4 carbon atoms,

$R^1$ and $R^5$, which are always different, represent an achiral straight-chain, branched or cyclic, saturated or unsaturated hydrocarbon radical with up to 8 carbon atoms, which is optionally interrupted in the chain by an oxygen or sulphur atom, and/or which is optionally substituted by halogen, such as fluorine or chlorine, cyano, alkoxy carbonyl with up to 4 carbon atoms in the alkyl part, phenyl, phenoxy, phenylthio or phenylsulphonyl, it being possible for the phenyl groups in turn to be substituted by halogen, such as fluorine or chlorine, cyano, dialkylamino with up to 4 carbon atoms per alkyl group, alkoxy or alkyl with in each case up to 4 carbon atoms, trifluoromethyl or nitro, or it being possible for the hydrocarbon radical to be substituted by pyridyl or an amino group, this amino group being substituted by two identical or different substituents from the group comprising alkyl with up to 4 carbon atoms, alkoxyalkyl with up to 6 carbon atoms, aryl, in particular phenyl, and aralkyl, in particular benzyl, or it being possible for the amino group to be substituted such that two radicals, together with the nitrogen atom, form a 5-membered to 7-membered heterocyclic ring, which can contain, as a further hetero-atom, oxygen or sulphur or an N-alkyl/phenyl grouping,

$R^2$ and $R^4$ can be identical or different and represent hydrogen or, preferably, a straightchain or branched, optionally substituted alkyl radical with up to 4 carbon atoms, a phenyl or a benzyl radical and

$R^3$ represents hydrogen or an achiral straightchain or branched alkyl radical with up to 4 carbon atoms, a phenyl radical or a benzyl radical,

characterised in that compounds of the general formula (II) (including their prototropic forms)

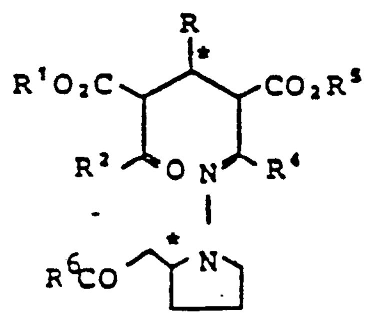

II

in which

the radicals R, $R^1$, $R^2$, $R^4$ and $R^5$ have the abovementioned meaning and

$R^6$ represents an alkyl or aralkyl radical, and in which the configuration at the carbon atoms designated (*) is defined as uniform,

are reacted with amines of the general formula (III)

$R^3\text{-}NH_2$ (III)

or acid addition salts thereof,
in which
$R^3$ has the abovementioned meaning, if appropriate in the presence of suitable solvents.

2. Process according to Claim 1, characterised in that acid addition salts of the compounds of the general formula (III) from the group comprising ammonium and methylammonium sulphates, chlorides, carbonates, bicarbonates, acetates and oxalates are used.

3. Process according to Claims 1 and 2, characterised in that the reaction is carried out at temperatures between 0 and 150° C in the presence of an inert organic solvent.

4. Process according to Claims 1 to 3, characterised in that the optical antipodes of the general formula (I) are purified by chromatography.

5. Compounds of the general formula (II)

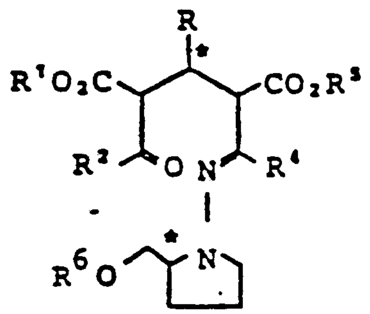

(II)

in which

R represents a phenyl or naphthyl radical, or represents thienyl, furyl, pyrryl, pyrazolyl, imidazolyl, oxazolyl, isoxazolyl, thiazolyl, pyridyl, pyridazinyl, pyrimidyl, pyrazinyl, indolyl, benzimidazolyl, benzoxazolyl, benzisoxazolyl, benzoxadiazolyl, benzothiadiazolyl, quinolyl, isoquinolyl, quinazolyl or quinoxalyl, it being possible for the heterocyclic radicals mentioned and the phenyl radical and naphthyl radical to carry 1 to 3 identical or different substituents, substituents which may be mentioned being phenyl, straight-chain or branched alkyl with up to 8 carbon atoms, cycloalkyl with 3 to 7 carbon atoms, alkenyl or alkinyl with 2 to 6 carbon atoms, alkoxy with 1 to 4 carbon atoms, alkenoxy and alkinoxy with 2 to 6 carbon atoms, tri-, tetra- and penta-methylene, dioxymethylene, dioxyethylidene, halogen, such as fluorine, chlorine, bromine or iodine, trifluoromethyl, trifluoroethyl, trifluoromethoxy, difluoromethoxy, tetrafluoroethoxy, dialkylamino with 1 to 4 C atoms, nitro, cyano, azido, alkoxycarbonyl with 1 to 4 C atoms in the alkoxy radical and $SO_m$-alkyl, m denoting 0 or 2 and alkyl containing 1 to 4 carbon atoms,

$R^1$ and $R^5$, which are always different, represent an achiral straight-chain, branched or cyclic, saturated or unsaturated hydrocarbon radical with up to 8 carbon atoms, which is optionally interrupted in the chain by an oxygen or sulphur atom, and/or which is optionally substituted by halogen, such as fluorine or chlorine, cyano, alkoxycarbonyl with up to 4 carbon atoms in the alkyl part, phenyl, phenoxy, phenylthio or phenylsulphonyl, it being possible for the phenyl groups in turn to be substituted by halogen, such as fluorine or chlorine, cyano, dialkylamino with up to 4 carbon atoms per alkyl group, alkoxy or alkyl with in each case up to 4 carbon atoms, trifluoromethyl or nitro, or it being possible for the hydrocarbon radical to be substituted by pyridyl or an amino group, this amino group being substituted by two identical or different substituents from the group comprising alkyl with up to 4 carbon atoms, alkoxyalkyl with up to 6 carbon atoms, aryl, in particular phenyl, and aralkyl, in particular benzyl, or it being possible for the amino group to be substituted such that two radicals, together with the nitrogen atom, form a 5-membered to 7-membered heterocyclic ring, which can contain, as a further hetero-atom, oxygen or sulphur or an N-alkyl phenyl grouping,

$R^2$ and $R^4$ can be identical or different and represent hydrogen or, preferably, a straightchain or branched, optionally substituted alkyl radical with up to 4 carbon atoms, a phenyl or a benzyl radical and

$R^6$ represents an achiral straight-chain or branched alkyl radical with up to 4 carbon atoms, in particular the methyl group, or an aralkyl radical, in particular the benzyl radical.

6. Process for the preparation of compounds of the general formula (II) according to Claim 5, characterised in that an optically active hydrazone of the general formula (IV)

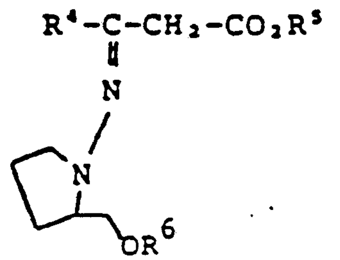

(IV)

in which
$R^4$, $R^5$ and $R^6$ have the meaning given in Claim 5, is reacted with stereochemically uniform prochiral ylidene-β-keto-carboxylic acid esters of the general formula (V)

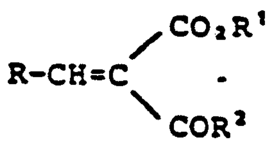

(V)

in which
R, $R^1$ and $R^2$ have the meaning given in Claim 5, if appropriate in the presence of inert organic solvents.

7. Use of compounds of the general formula (II) according to Claim 5 in the preparation of compounds of the general formula (I) according to Claim 1.

**Revendications**

1. Procédé de production d'antipodes d'esters d'acides 1,4-dihydropyridine-dicarboxyliques chiraux de formule générale (I)

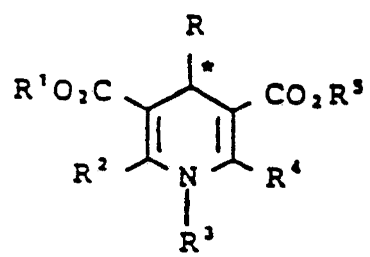

(I)

dans laquelle
R est un reste phényle ou naphtyle ou un reste thiényle, furyle, pyrryle, pyrazolyle, imidazolyle, oxazolyle, isoxazolyle, thiazolyle, pyridyle, pyridazinyle, pyrimidyle, pyrazinyle, indolyle, benzimidazolyle, benzoxazolyle, benzisoxazolyle, benzoxadiazolyle, benzothiadiazolyle, quinolyle, isoquinolyle, quinazolyle ou quinoxalyle, les hétérocycles mentionnés de même que le reste phényle et le reste naphtyle pouvant porter 1 à 3 substituants identiques ou différents tels que phényle, alkyle à chaîne droite ou à chaîne ramifiée ayant jusqu'à 8 atomes de carbone, cycloalkyle ayant 3 à 7 atomes de carbone, alcényle ou alcynyle ayant 2 à 6 atomes de carbone, alkoxy ayant 1 à 4 atomes de carbone, alcénoxy ou alcynoxy ayant 2 à 6 atomes de carbone, tri-, tétra- et pentaméthylène, dioxyméthylène, dioxyéthylidène, halogène tel que fluor, chlore, brome ou iode, trifluorométhyle, trifluoréthyle, trifluorométhoxy, difluorométhoxy, tétrafluoréthoxy, dialkylamino ayant 1 à 4 atomes de carbone, nitro, cyano, azido, alkoxycarbonyle ayant 1 à 4 atomes de carbone dans le reste alkoxy, ou $SO_m$-alkyle, où m a la valeur 0 ou 2 et alkyle contient 1 à 4 atomes de carbone,
$R^1$ et $R^5$, qui sont toujours différents, représentent un reste hydrocarboné achiral saturé ou non saturé, à chaîne droite, à chaîne ramifiée ou cyclique, ayant jusqu'à 8 atomes de carbone, qui est interrompu éventuellement dans la chaîne par un atome d'oxygène ou de soufre et/ou qui est éventuellement substitué par un halogène tel que fluor ou chlore, un groupe cyano, alkoxycarbonyle ayant jusqu'à 4 atomes de carbone dans la partie alkyle, phényle, phénoxy, phénylthio ou phénylsulfonyle, les groupes phényle pouvant quant à eux être substitués par un halogène tel que fluor ou chlore, un groupe cyano,

dialkylamino ayant jusqu'à 4 atomes de carbone par groupe alkyle, alkoxy ou alkyle ayant chacun jusqu'à 4 atomes de carbone, trifluorométhyle ou nitro, ou bien le reste hydrocarboné peut être substitué par un groupe pyridyle ou par un groupe amino, ce groupe amino étant substitué par deux substituants identiques ou différents du groupe des substituants alkyle ayant jusqu'à 4 atomes de carbone, alkoxyalkyle ayant jusqu'à 6 atomes de carbone, aryle, notamment phényle, ou aralkyle, notamment benzyle, ou bien le groupe amino est substitué de telle manière que deux restes forment conjointement avec l'atome d'azote un noyau hétérocyclique pentagonal à heptagonal qui peut contenir comme autre hétéroatome de l'oxygène ou du soufre ou un groupement N-alkyle/phényle,

$R^2$ et $R^4$ peuvent être identiques ou différents et representent l'hydrogène ou de préférence un reste alkyle à chaîne droite ou ramifié, éventuellement substitué, ayant jusqu'à 4 atomes de carbone, un reste phényle ou un reste benzyle, et

$R^3$ représente l'hydrogène ou un reste alkyle achiral à chaîne droite ou à chaîne ramifiée ayant jusqu'à 4 atomes de carbone, un reste phényle ou un reste benzyle,

caractérisé en ce qu'on fait réagir des composés de formule générale (II) (y compris leurs formes prototropes)

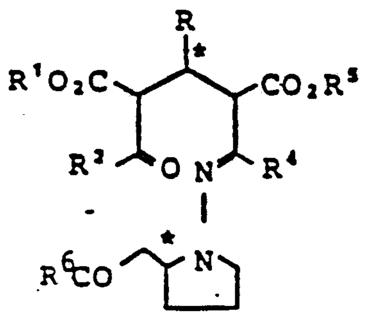

II

dans laquelle

les restes R, $R^1$, $R^2$, $R^4$ et $R^5$ ont la définition indiquée ci-dessus et

$R^6$ représente un reste alkyle ou aralkyle et dans laquelle la configuration au niveau des atomes de carbone désignés par un astérisque (*) est nettement définie,

avec des amines de formule générale (III)

$R^3$-$NH_2$ (III)

ou leurs sels d'addition d'acides,

formule dans laquelle $R^3$ a la définition indiquée ci-dessus,

le cas échéant en présence de solvants appropriés.

2. Procédé suivant la revendication 1, caractérisé en ce qu'on utilise des sels d'addition d'acides des composés de formule générale (III) du groupe des sulfates, chlorures, carbonates, hydrogénocarbonates, acétates ou oxalates d'ammonium et de méthylammonium.

3. Procédé suivant les revendications 1 et 2, caractérisé en ce qu'on conduit la réaction à des températures comprises entre 0 et 150° C et en présence d'un solvant organique inerte.

4. Procédé suivant les revendications 1 à 3, caractérisé en ce qu'on purifie par chromatographie les antipodes optiques de formule générale (1).

5. Composés de formule générale (II)

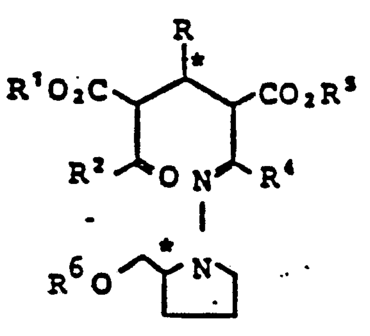

(II)

dans laquelle

R représente un reste phényle ou naphtyle ou un reste thiényle, furyle, pyrryle, pyrazolyle, imidazolyle,

oxazolyle, isoxazolyle, thiazolyle, pyridyle, pyridazinyle, pyrimidyle, pyrazinyle, indolyle, benzimidazolyle, benzoxazolyle, benzisoxazolyle, benzoxadiazolyle, benzothiadiazolyle, quinolyle, isoquinolyle, quinazolyle ou quinoxalyle, les hétérocycles mentionnés de même que le reste phényle et le reste naphtyle pouvant porter 1 à 3 substituants identiques ou différents, tels que des substituants phényle, alkyle à chaîne droite ou ramifié ayant jusqu'à 8 atomes de carbone, cycloalkyle de 3 à 7 atomes de carbone, alcényle ou alcynyle de 2 à 6 atomes de carbone, alkoxy de 1 à 4 atomes de carbone, alcénoxy et alcynoxy de 2 à 6 atomes de carbone, triméthylène, tétraméthylène et pentaméthylène, dioxyméthylène, dioxyéthylidène, un halogène tel que le fluor, le chlore, le brome ou l'iode, un reste trifluorométhyle, trifluoréthyle, trifluorométhoxy, difluorométhoxy, tétrafluoréthoxy, dialkylamino de 1 à 4 atomes de carbone, nitro, cyano, azido, alkoxycarbonyle ayant 1 à 4 atomes de carbone dans le reste alkoxy ou $SO_m$-alkyle où m a la valeur 0 ou 2 et alkyle comprend 1 à 4 atomes de carbone,

$R^1$ et $R^5$, qui sont toujours différents, représentent un reste hydrocarboné achiral saturé ou non saturé, à chaîne droite, à chaîne ramifiée ou cyclique ayant jusqu'à 8 atomes de carbone, qui est éventuellement interrompu par un atome d'oxygène ou de soufre dans la chaîne et/ou qui est éventuellement substitué par un halogène tel que le fluor ou le chlore, un groupe cyano, alkoxycarbonyle ayant jusqu'à 4 atomes de carbone dans la partie alkyle, phényle, phénoxy, phénylthio ou phénylsulfonyle, les groupes phényle pouvant être substitués quant à eux par un halogène tel que le fluor ou le chlore, un groupe cyano, dialkylamino ayant jusqu'à 4 atomes de carbone par groupe alkyle, alkoxy ou alkyle ayant chacun jusqu'à 4 atomes de carbone, trifluorométhyle ou nitro, le reste hydrocarboné pouvant être substitué par un groupe pyridyle ou un groupe amino, ce groupe amino étant substitué par deux substituants identiques ou différents du groupe des substituants alkyle ayant jusqu'à 4 atomes de carbone, alkoxyalkyle ayant jusqu'à 6 atomes de carbone, aryle, notamment phényle, ou aralkyle, notamment benzyle, ou bien le reste amino étant substitué de telle manière que deux restes forment conjointement avec l'atome d'azote un noyau hétérocyclique pentagonal à heptagonal qui peut contenir comme autre hétéroatome de l'oxygène ou du soufre ou un groupement N-alkyle/phényle,

$R^2$ et $R^4$, qui peuvent être identiques ou différents, représentent de l'hydrogène ou de préférence un reste alkyle à chaîne droite ou à chaîne ramifiée, éventuellement substitué, ayant jusqu'à 4 atomes de carbone, un reste phényle ou un reste benzyle, et

$R^6$ représente un reste alkyle achiral à chaîne droite ou à chaîne ramifiée ayant jusqu'à 4 atomes de carbone, notamment le groupe méthyle ou un reste aralkyle, notamment le reste benzyle.

6. Procédé de préparation de composés de formule générale (II) suivant la revendication 5, caractérisé en ce qu'on fait réagir, éventuellement en présence de solvants organiques inertes, une hydrazone optiquement active de formule générale (IV)

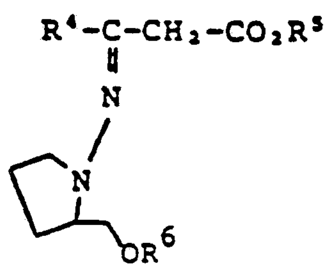

(IV)

dans laquelle

$R^4$, $R^5$ et $R^6$ ont la définition indiquée dans la revendication 6,

avec des esters d'acides ylidène-β-cétocarboxyliques prochiraux bien définis du point de vue stéréochimique, de formule générale (V)

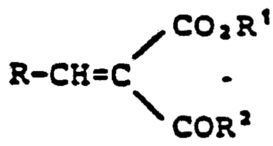

(V)

dans laquelle

R, $R^1$ et $R^2$ ont la définition indiquée dans la revendication 6.

7. Utilisation de composés de formule générale (II) suivant la revendication 5, dans la préparation de composés de formule générale (I) suivant la revendication 1.